# EUROPÄISCHE PATENTSCHRIFT

(11) **EP 3 263 154 B1**
(45) Veröffentlichungstag und Bekanntmachung des Hinweises auf die Patenterteilung: **17.07.2019**
(21) Anmeldenummer: 17184739.5
(22) Anmeldetag: 29.11.2013
(51) Int. Cl.: A61M 1/36, B01D 36/00, B01D 63/02, F16K 24/04

(54) **VORRICHTUNG ZUR RASCHEN ENTLÜFTUNG UND ENTLEERUNG EINES FILTERS**
DEVICE FOR QUICKLY VENTING AND DRAINING A FILTER
DISPOSITIF DE PURGE D'AIR ET DE VIDANGE RAPIDE D'UN FILTRE

(30) Priorität: 03.12.2012 DE 102012023504; 03.12.2012 US 201261732628 P
(43) Veröffentlichungstag der Anmeldung: 03.01.2018
(62) Teilanmeldung aus: 13798660.0
(73) Patentinhaber: Fresenius Medical Care Deutschland GmbH, 61352 Bad Homburg (DE)
(72) Erfinder: Konegger, Mario, 6020 Innsbruck (AT)
(74) Vertreter: Dreyhsig, Jörg

(56) Entgegenhaltungen:
- DE-C1- 4 027 531
- DE-U1- 8 603 781
- US-A- 4 104 004
- US-A- 4 320 001
- US-A- 4 981 154
- US-A1- 2011 108 482

## Beschreibung

Die Erfindung betrifft eine Vorrichtung zur Entlüftung eines Filters, bevorzugt eines Hohlfaserfilters, nach dem Oberbegriff des Anspruchs 1.

Filter werden in vielen weiten Bereichen der Industrie und in der Medizin zur Reinigung von Gasen und Flüssigkeiten eingesetzt. Die eingesetzten Filter sind dabei derart gestaltet, dass sie aus zwei Kammern bestehen, die voneinander mit einer semipermeablen Membran getrennt sind. Die semipermeable Membran ist dabei derart beschaffen, dass durch sie aus einer ersten Kammer Flüssigkeit in eine zweite Kammer gedrückt werden kann, während höhermolekulare Stoffe zurückgehalten werden.

Ein Anwendungsgebiet von Fluidfiltern sind Verfahren zur Fluidbehandlung oder Blutbehandlung, etwa der Hämodialyse oder der Peritonealdialyse.

Bei der Hämodialyse wird Blut in einem extrakorporalen Kreislauf kontinuierlich einem Patienten entnommen, durch einen Hämodialysator geleitet und dem Patienten wieder reinfundiert. Dabei wird ein Stoffaustausch durchgeführt, der dem der Nieren ähnlich ist. Der Hämodialysator besteht aus zwei durch eine semipermeable Membran getrennte Kammern, von denen die eine vom Blut und die andere von einer Reinigungsflüssigkeit - der Dialysierflüssigkeit - durchflossen wird. Die handelsüblichen Hämodialysatoren weisen hierfür meist Tausende von Hohlfasern auf, deren Wände die semipermeable Membran für die auszutauschenden Substanzen bilden. Das Blut wird durch den Innenraum der Hohlfasern geleitet, während die Dialysierflüssigkeit in meist gegenläufiger Richtung in den Hohlfaserzwischenraum eingespeist und abgeführt wird.

Die Dialysierflüssigkeit weist Konzentrationen von Blutinhaltsstoffen wie Elektrolyten auf, die in etwa denen eines gesunden Menschen entsprechen, damit die entsprechenden Konzentrationen im Blut auf einem normalen Niveau gehalten werden können. Aus dem Blut zu entfernende Stoffe wie zum Beispiel Kreatinin oder Harnstoff sind in der Dialysierflüssigkeit nicht enthalten, wodurch diese allein wegen des Konzentrationsgradienten an der Membran durch Diffusion aus dem Blut entfernt werden. Mit Hilfe eines Druckgradienten wird dem Blut überschüssiges Wasser durch Konvektion beziehungsweise Ultrafiltration entzogen. Der aus Konvektion und Diffusion kombinierte Entzug wird als Diafiltration bezeichnet.

Zur Steuerung derartiger Vorgänge werden Hämodialysegeräte eingesetzt, die zumeist auch die Zubereitung der Dialysierflüssigkeit aus Wasser und Konzentraten mit der richtigen Zusammensetzung und Temperatur sicherstellen. Bei der Hämodiafiltration wird dem Blut des Patienten während einer Hämodialysebehandlung über den Hämodialysator eine größere Menge Ultrafiltrat entzogen, die bis auf die insgesamt zu entziehende Flüssigkeitsmenge wieder durch Substitutionsflüssigkeit ersetzt wird. Bei modernen Geräten zur Behandlung des chronischen Nierenversagens wird hierfür die online aufbereitete Dialysierflüssigkeit verwendet, indem eine von dem Dialysierflüssigkeitskreislauf abzweigende Leitung mit einem oder mehreren Filterstufen versehen und mit dem extrakorporalen Blutkreislauf stromauf und/oder stromab des Hämodialysators verbunden wird. Die Zugabe der zusätzlich gefilterten Dialysierflüssigkeit in den Blutkreislauf wird als Dilution bezeichnet.

Zu Beginn der Dialysebehandlung müssen die in den Filterstufen angeordneten Filter mit Dialysierflüssigkeit durchspült und entlüftet werden.

Aus der DE 4027531 C1 ist eine Anordnung bekannt, die mittels eines Hydrophobfilter an einem oberen Ende eines Hohlfaserfilters eine Entlüftung erreicht. Bei dieser Anordnung ist nachteilig, dass sich das Hydrophobfilter über den gesamten Querschnitt des Hohlfaserfilters erstreckt und somit nicht Druckstabil ist. Des Weiteren ist eine derartige Membran nachteilig bezüglich der Herstellungskosten.

Aus der DE 3444671 A1 ist ein Verfahren bekannt, welches eine Entlüftung eines Filters mittels Sterilfilter und Hydrophobfilter erreicht. Der in zwei Kammern aufgeteilte zu entlüftende Filter hat dabei in der ersten Kammer an deren unteren Ende einen für die Zufuhr von Flüssigkeit vorgesehenen Anschluss, und am oberen Ende einen Anschluss mit einer Ausgleichskammer und dem besagten Hydrophobfilter. Die zweite Kammer hat am unteren Ende einen Anschluss, der mit dem besagten Sterilfilter verbunden ist und die für das Entlüften über dem besagten Hydrophobfilter angeordnet wird. Das Entlüften erfolgt nun derart, dass über den unteren Anschluss der ersten Kammer Flüssigkeit eingebracht wird und die in dieser Kammer befindliche Luft durch das Hydrophobfilter verdrängt. Sobald die gesamte Luft verdrängt ist, wird der Hydrophobfilter mit Flüssigkeit beaufschlagt und damit für weiteren Durchgang für Luft undurchlässig. Nun wird die Flüssigkeit durch die Membran des zu entlüftenden Filters gedrückt und beginnt die Luft in der zweiten Kammer durch das Sterilfilter zu verdrängen. Sobald im Wesentlichen keine Luft mehr durch das Sterilfilter entweicht, ist der Entlüftungsvorgang abgeschlossen. Nachteilig bei diesem Verfahren ist, dass das Sterilfilter nur einmalig verwendet werden kann.

Weiter nachteilig ist dabei, dass das Hydrophobfilter mit Flüssigkeit benetzt wird und somit die Sterilität und Druckfestigkeit fortan nicht mehr gewährleistet ist.

Wenn die Sterilität beeinträchtigt ist besteht die unmittelbare Gefahr, dass Verunreinigungen oder Keime wie Pilze und Bakterien von der Substitutionsleitung in das Blut des Patienten übergehen.

Auch nachteilig ist die mehrmalige notwendige manuelle Intervention von seitens des bedienenden Personals.

Aus WO 2006/049822 ist ein Verfahren zur Entlüftung eines Filters bekannt, bei dem der Filter aus der vertikalen Position heraus etwas geneigt wird. Damit wird erreicht, dass sich Luft am oberen Ende des Filters an der so entstandenen höchsten Stelle sammelt. Die Entlüftung kann nun über diese höchste Stelle erfolgen, wobei hier jedoch nachteilig eine manuell zu verschließende Klemme nach Entlüftung geschlossen werden muss.

Es ist bekannt, dass Hydrophobfilter Gase oder Gasgemische, insbesondere Luft und Dampf, durchlassen, nicht jedoch Flüssigkeiten und sonstige Feststoffe wie beispielsweise Bakterien und Toxine. Aufgrund dieser Eigenschaft werden Hydrophobfilter als Sterilfilter eingesetzt. Kommt die Membran des Hydrophobfilters mit Flüssigkeit in Berührung, so verliert sie die Eigenschaft der Gas- und Dampfdurchlässigkeit auch vollständig, so dass keinerlei Durchlässigkeit mehr gegeben ist. Darüber hinaus könne sich Bakterien an feuchte Stellen ansetzen und so an die feuchte Hydrophobmembran gelangen.

In einem Dialysierflüssigkeitskreislauf mit einem Hydrophobfilter und einem diesen abschließenden Anschluss muss der Anschluss nicht notwendig verschlossen werden, da das Hydrophobfilter druckbeständig ist und während der Behandlung normalerweise einen gegenüber dem Umgebungsdruck erhöhten Druck aufweist. Es ist jedoch vorteilhaft, das Hydrophobfilter nicht während der Behandlung alleinig als Druckbarriere zu benutzen. Dies ist insbesondere deshalb nachteilig, da während der Behandlung Druckschwankungen, hervorgerufen unter anderem durch Pumpen im Dialysierflüssigkeitskreislauf, eine wechselnde Druckbelastung an der Hydrophobfiltermembran hervorrufen und das Risiko des Brechens und damit verbunden ungewollten Flüssigkeitsaustritt und Probleme der Dilution am zu behandelnden Patient nach sich ziehen kann. Des Weiteren kann eine Hydrophobmembran so ihre Wirkung als Sterilfilter verlieren, so dass während der Behandlung ein erhöhtes Risiko für eine Kontamination des blutseitigen Kreislaufs besteht.

Die US 4 320 001 A offenbart einen Blutfilter mit einem Entlüftungsventil, wobei das Ventil folgende Merkmale umfasst: einen Körper mit einem Hohlraum, einer ersten Öffnung und einer zweiten Öffnung, ein erstes Abschlusselement, das mit dem Körper verbunden ist und die zweite Öffnung aufweist, in der eine Bakterienbarriere aufgenommen ist, ein kugelförmiges Dichtelement im Inneren des Hohlraums, das derart ausgebildet ist, um in einer Flüssigkeit an der Oberfläche zu schwimmen und das zwischen einer unteren Position und einer oberen Position bewegbar ist, ein Abschlusselement, welches mit dem Körper verbunden ist und die untere Position vorgibt und ein Begrenzungselement, welches die obere Position vorgibt und eine Öffnung aufweist.

### Zusammenfassung

Der Erfindung liegt daher die Aufgabe zugrunde zumindest einen der genannten Nachteile zu überwinden sowie ein Entlüftungsventil und einen Filter mit einem zugehörigen Entlüftungsventil anzugeben, die eine automatische Entlüftung ermöglicht.

Diese Aufgabe wird mit dem Filter mit Entlüftungsventil nach Anspruch 1 gelöst.

Vorteilhafte Weiterbildungen der Vorrichtung nach Anspruch 1 sind in den abhängigen Ansprüchen angegeben.

Offenbart wird ein Entlüftungsventil zum Entlüften eines Filters, vorzugsweise eines Filters für eine Blutbehandlungsmaschine etwa eines Filters für einen Dialysierflüssigkeitskreislauf einer Dialysemaschine. Das Entlüftungsventil umfasst einen Hohlraum mit einer ersten und einer zweiten Öffnung und einem Dichtelement im Innern des Hohlraums zum Abdichten der ersten Öffnung in einer ersten Position und zum Abdichten der zweiten Öffnung in einer zweiten Position. Das Dichtelement ist zwischen der ersten und der zweiten Position frei beweglich so dass ein Durchfluss zwischen der ersten und der zweiten Öffnung gebildet wird, wenn das Dichtelement sich in einer Zwischenposition zwischen der ersten und der zweiten Position befindet.

In einer Ausführungsform werden im Betrieb der Dialysemaschine und somit im Betrieb des Dialysierflüssigkeitskreislaufs eine senkrechte und eine horizontale Richtung sowie eine untere und eine obere Position vorgeben und das Dichtelement ist im Betrieb der Dialysemaschine und des Dialysierflüssigkeitskreislaufs so zwischen der oberen und der unteren Position beweglich, dass die Bewegungstrajektorie zwischen der unteren und der oberen Position eine Komponente in senkrechter Richtung aufweist. Mit anderen Worten: die erste Position ist eine untere Position und die zweite Position ist eine obere Position.

Weiterhin vorteilhaft ist das Dichtelement als Schwimmkörper zum Schwimmen auf der Dialysierflüssigkeit ausgebildet. Auf diese Weise trägt eine Auftriebskraft mit senkrechter, d.h. nach oben gerichteter Komponente zur Abdichtung des Dichtelements in der oberen Position bei.

In einer Ausführungsform umfasst die Oberflache des Dichtelements ein hydrophobes Material. Es kann aber auch das gesamte Dichtelement aus hydrophobem Material bestehen.

In der Anwendung im Zusammenhang mit einer Blutbehandlung, und/ oder einer Blutbehandlungsmaschine besteht ein Vorteil der offenbarten Vorrichtung darin, dass die Handhabung und die Fehlertoleranz der Bedienung verbessert und/ oder die notwendigen manuell auszuführenden Schritte des bedienenden Personals verringert. Weiterhin sind die Kosten für die Herstellung gemindert.

### Kurzbeschreibung der Zeichnungen

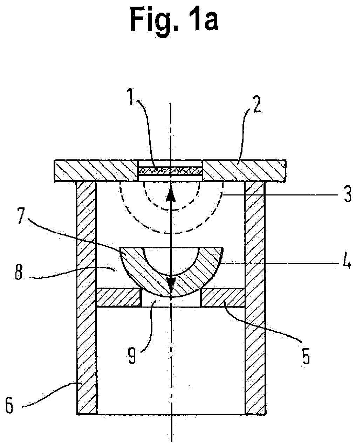

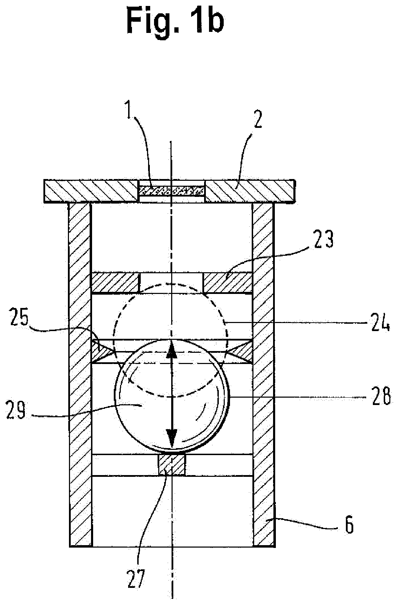

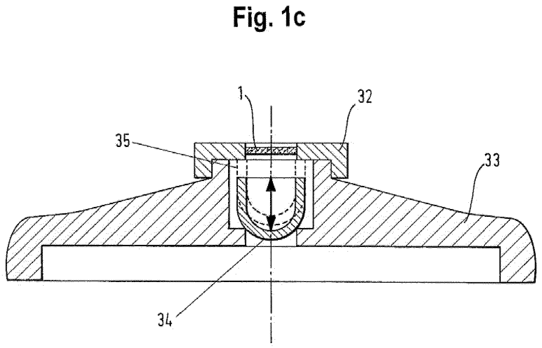

Figur 1 a zeigt ein Entlüftungsventil, welches nicht Teil der Lehre der vorliegenden Erfindung ist.
Figur 1 b zeigt eine Ausgestaltung eines erfindungsgemäßen Entlüftungsventils.
Figur 1 c zeigt die Integration eines nicht erfindungsgemäßen Entlüftungsventils in ein Gehäuse einer Filtervorrichtung.

### Detaillierte Beschreibung der Zeichnungen

Im Folgenden werden die Vorrichtung im Einklang mit der erfindungsgemäßen Lehre und vorteilhafte Weiterbildungen anhand der Abbildungen 1a, 1b und 1c näher erläutert, ohne dass die erfindungsgemäße Lehre darauf eingeschränkt sein soll, wobei die Figur 1b eine erfindungsgemäße Ausführung zeigt und die Figuren 1a und 1c nicht erfindungsgemäße Ausführungsformen zeigen.

Abbildung 1a zeigt eine nicht erfindungsgemäße Ausführung eines Entlüftungsventils für einen Dialysierflüssigkeitskreislauf einer Dialysemaschine mit einer Hydrophobmembran 1, die vorteilhaft zugleich als Sterilfilter wirkt, mit einem Abschlusselement 2, das vorteilhaft als Aufnahmevorrichtung 2 für die Aufnahme der Hydrophobmembran 1 ausgebildet ist. Abschlusselement 2 ist mit dem bevorzugt innen zylindrisch geformten Körper 6, der nach beiden Richtungen hin offen ist und einen rohrförmigen Hohlraum bildet, verbunden und schließt diesen an einem ersten Ende vollständig ab. Am zweiten Ende ist der zylindrische Innenraum des Körpers 6 mittels des Abschlusselements 5 mit einer Öffnung 9, welches bevorzugt als ein scheibenförmiger Körper mit einem zentrisch axial verlaufenden angeordneten Loch ausgebildet ist, verbunden. Das Abschlusselement 5 schließt den Innenraum des Körpers 6 nach unten ab. In einer weiteren Ausgestaltung kann der Körper 6 auch bereits das Abschlusselement 5 mit ausformen. Im entstehenden Hohlraum 8 im inneren des Körpers 6 befindet sich ein beweglich angeordnetes Dichtelement 7, das so geformt ist, dass er durch Andrücken an das Abschlusselement 2 oder das Abschlusselement 5 einen gas-, dampf- und/oder flüssigkeitsdichten Verschluss bildet, wobei das Dichtelement 7 jeweils nur einen oder eines der beiden Abschlusselemente nicht jedoch beide zugleich verschließen kann. Im Betrieb der Dialysemaschine sind eine horizontale und eine vertikale Orientierung definiert, sowie eine obere Position 3 des Dichtelements 7 und eine untere Position 4 des Dichtelements 7. Im Betrieb der Dialysemaschine ist das Dichtelement 7 zwischen der unteren Position 4 und der oberen Position 3 beweglich. Die Bewegungstrajektorie zwischen der unteren Position 4 und der oberen Position 3 enthält somit zumindest eine Komponente in vertikaler Richtung. Um eine Bewegung des Dichtelements 7 entlang der Bewegungstrajektorie sicherzustellen können Führungsmittel (nicht gezeigt) vorgesehen sein. Das Dichtelement 7 ist vorzugsweise als Schwimmkörper zum Aufschwimmen auf einer Flüssigkeit ausgebildet, in der Anwendung in einem Dialysierflüssigkeitskreislauf: zum Aufschwimmen auf der Dialysierflüssigkeit.

Steigt in dem Körper 6 Flüssigkeit von unten nach oben an, so ist das Dichtelement 7 zunächst in Folge des Gewichts in der unteren Position 4 und an das Abschlusselement 5 vollständig verschließend aufliegend. Flüssigkeit und/oder das Gas und/oder der Dampf hebt anschließend das schwimmend ausgeführte Dichtelement 7 an und ermöglicht so, dass Gas durch die Membran des Hydrophobfilters 1 entweichen kann, bis das Dichtelement 7 in der Position 3 an das Abschlusselement 2 angedrückt wird und diese verschließt. Ein weiteres Austreten von Flüssigkeit, Dampf, und/oder Gas ist dann nicht mehr möglich. Bei der Ausführung des Dichtelements 7 als Schwimmkörper wirkt eine Auftriebskraft auf das Dichtelements 7, die der oberen Position 3 als Dichtkraft wirkt.

Vorteilhaft bei dieser Anordnung ist dabei, dass ein in einem angeschlossenen Fluidkreislauf durchgeführter Druckhaltetest keine Beschädigung der Hydrophobmembran verursacht.

Weiterhin vorteilhaft ist, dass eine kostengünstige Herstellung ermöglicht wird.

Weiterhin vorteilhaft im Zusammenhang mit der Anwendung des Entlüftungsventils zur Entlüftung eines Filters in einem Fluidkreislauf einer Blutbehandlungsmaschine, insbesondere in einer Dialysemaschine ist es, dass durch das Verschließen des Abschlusselements 5 in der unteren Position 4 kein Eindringen von Luft während des Betriebes der Blutbehandlung infolge von Druckschwankungen hervorgerufen werden kann und dass so keine Luft durch Pumpen zurück in den Filter gelangen kann.

Das Dichtelement 7 ist vorteilhaft aus elastischem Kunststoff ausgeführt, wobei die Oberfläche in vorteilhafter Weise aus einem hydrophoben Material besteht, oder mit diesem beschichtet ist. Damit wird erreicht, dass das Dichtelement 7 in Position 3 zusätzlich zur Auftriebskraft noch eine Kraft hervorgerufen durch die Oberflächenspannung erfährt und somit den Anpressdruck des Dichtelements 7 an die Aufnahmevorrichtung 2 und so die Dichtigkeit in Position 3 unterstützt.

Das Gewicht des Dichtelements 7 ist vorteilhaft so gewählt, dass bei Abwesenheit von Flüssigkeit in dem Körper 6 auf Grund der Gewichtskraft des Dichtelements 7 eine zum Abdichten in der unteren Position 4 ausreichende Dichtkraft vorhanden ist.

Abbildung 1b zeigt eine Ausgestaltung der Erfindung, welche in Zusammenhang mit der nicht erfindungsgemäßen, in Figur 1a beschriebenen Vorrichtung beschrieben wird. Gleiche oder entsprechende Elemente sind mit gleichen Bezugszeichen wie in Figur 1a versehen, wobei auf die Beschreibung der Figur 1a Bezug genommen wird an Stelle einer Wiederholung. An die Stelle des Dichtelements 7 tritt ein kugelförmiges Dichtelement 29, das sich in einem bevorzugt zylindrischen Hohlraum des Körpers 6 im Wesentlichen auf und ab bewegen kann zwischen einer unteren Position 28, vorgegeben durch das Abschlusselement 27 und einer oberen Position 24, vorgegeben durch das Abschlusselement 2 oder in einer vorteilhaften Weiterbildung durch das zusätzlich angebrachte Begrenzungselement 23. Die Hydrophobmembran 1, das Abschlusselement 2, und der bevorzugt zylindrisch ausgeführte Körper 6 entsprechen den im Zusammenhang mit Figur 1a beschriebenen Elemente. Zusätzlich weist die Anordnung von Figur 1b ein mit dem bevorzugt zylindrisch ausgeführten Körper 6 verbundenes Abschlusselement 27 auf. Die Abschlusselemente 2 und 23 können mit dem Körper 6 einstückig ausgeführt sein.

Ein Halteelement 25 ist zwischen dem Begrenzungselement 23 und dem Abschlusselement 27 derart angeordnet, dass, wenn sich das Dichtelement 29 in der Position 24 befindet, dieses die Öffnung in dem Begrenzungselement 23 und in dem Halteelement 25 vollständig gas-, dampf, und/oder flüssigkeitsfest verschließt und zusätzlich von dem Halteelement 25 in Position 24 gehalten wird, jedoch in der unteren Position 28 einen Durchfluss für Gase und Flüssigkeiten ausbildet. Das Halteelement 25 ist derart gestaltet, dass das Dichtelement 29 nur durch Beaufschlagung einer hinreichend großen Kraft von Position 24 nach Position 28 beziehungsweise von Position 28 nach Position 24 verschoben werden kann. Die Beaufschlagung der Kraft erfolgt vorteilhaft durch Druck, aufgebaut beispielsweise durch eine Pumpe in einem an das Entlüftungsventil abgeschlossenen Fluidkreislaufs. Bei der Entlüftung eines an das Entlüftungsventil angeschlossenen Filters wird zunächst Luft durch den Körper 6 von unten nach oben austreten und erst wenn Flüssigkeit das auf der Flüssigkeit aufschwimmende Dichtelement 9, soweit anhebt, dass es mit dem Dichtelement 25 gas- und flüssigkeitsdicht verschließt, kann über die Zufuhr weiterer Flüssigkeit der nötige Druck und damit die nötige Kraft aufgebaut werden, um das bewegliche Dichtelement 29 in Position 24 zu bewegen. Umgekehrt kann durch Erzeugung eines entsprechend hohen Unterdruckes das Dichtelement 29 von der Position 24 in die Position 28 bewegt werden und somit eine durchgängige Verbindung in dem Körper 6 hergestellt werden. Der bewegliche Dichtelement 29 und/oder das Haltelement 25 sind bevorzugt aus einem elastischen Material, beispielsweise weichem Kunststoff, Gummi oder Ähnlichem auszuführen. Der erforderliche Druck, um das Dichtelement 29 in die Position 24 zu bringen ist etwa durch die Wahl des Materials und der Abmessung des Dichtelements 29 und des Durchmessers der in dem Halteelement 25 vorgesehenen Öffnung vorteilhaft derart bemessen, dass er größer ist als die Druckschwankungen die während des Betriebs eines an das Entlüftungsventil angeschlossenen Fluidkreislaufs, etwa eines Dialysatkreislaufs einer Blutbehandlungsmaschine auftreten können. So kann ein versehentliches Öffnen verbunden mit ungewolltem Lufteintritt vermieden werden. Andererseits sollte der erforderliche Druck geringer als der maximal durch die angeschlossene Vorrichtung erzeugbare Druck sein. Damit kann auf vorteilhafte Weise erreicht werden, dass die Vorrichtung automatisch durch das Gerät ohne zusätzliche Vorrichtungen an der Gerätevorderseite bedient werden kann, wenn das Entlüftungsventil auf der Geräterückseite angeordnet ist. Weiters vorteilhaft ist, dass das Ventil wieder geöffnet werden kann und damit bei der Anwendung in einem Dialysatkreislauf einer Dialysemaschine nach der Behandlung und der Re-Infusion ein rascheres und rückstandfreieres Entleeren erfolgt.

Abbildung 1c zeigt in einer vorteilhaften Ausgestaltung eine vorteilhafte Integration der Vorrichtung entsprechend der Abbildung 1a, in einer Variante, in das Gehäuse der Filtervorrichtung, bevorzugt in einen Deckel eines Filtergehäuses.

In ähnlicher Weise wie in den zuvor beschriebenen Vorrichtungen, weist diese Vorrichtung der Figur 1c eine Hydrophobmembran 1 die zugleich ein Sterilfilter sein kann, ein als Vorrichtung für die Aufnahme der Hydrophobmembran 1 ausgebildetes Abschlusselement 32, welches mit dem bevorzugt innen zylindrisch geformten Grundkörper 33, der zugleich auch bevorzugt den Deckel eines Filtergehäuses darstellt, der nach beiden Richtungen hin offen ist, verbunden ist und diesen an einem ersten Ende, bevorzugt dem oberen Ende, vollständig abschließt. Das bewegliche Dichtelement in der Abbildung 1c in Position 34 eingezeichnet, kann sich jedoch entlang des bevorzugt zylindrischen Hohlraumes in dem Grundkörper 33 im Wesentlichen entlang einer bevorzugt senkrecht verlaufenden Achse von Position 34 bis Position 35 bewegen. Die beiden Positionen sind dabei derart vorgegeben, dass in Position 35 der bewegliche Teil unter Anpressung an das Abschlusselement 32 einen luft-, dampf- und/oder flüssigkeitsdichten Verschluss bildet und in Position 34 das bewegliche Dichtelement einen luft-, dampf- und/oder flüssigkeitsdichten Verschluss unter Anpressung an das Abschlusselement 32 bildet. Die Kraft mit der der bewegliche Dichtelement 32 angepresst wird, ergibt sich in Position 35 aus der Druckdifferenz zwischen Innen- und Aussenraum, sowie der Auftriebskraft des aufschwimmenden Dichtelements abzüglich der Gewichtskraft, in Position 34 ergibt sich die Anpresskraft aus der Gewichtskraft und der Druckdifferenz.

## Patentansprüche

1. Filter für einen Dialysierflüssigkeitskreislauf, wobei der Filter ein Entlüftungsventil zum Entlüften des Filters aufweist, und wobei das Ventil umfasst
o einen Körper (6) mit einem Hohlraum (8), einer ersten (9) Öffnung und einer zweiten Öffnung (1),
o ein erstes Abschlusselement (2), das mit dem Körper (6) verbunden ist und die zweite Öffnung (1) aufweist, in der eine Hydrophobmembran aufgenommen ist,
o ein kugelförmiges Dichtelement (29) im Inneren des Hohlraums, das derart ausgebildet ist, um in einer Flüssigkeit an der Oberfläche zu schwimmen und das zwischen einer unteren Position (28) und einer oberen Position (24) bewegbar ist,
o ein Abschlusselement (27), welches mit dem Körper (6) verbunden ist und die untere Position (28) vorgibt,
o ein Begrenzungselement (23), welches die obere Position (22) vorgibt und eine Öffnung aufweist,
o ein Halteelement (25) mit einer Öffnung, welches derart zwischen dem Abschlusselement (27) und dem Begrenzungselement (23) angeordnet ist, dass, wenn sich das Dichtelement (29) in der oberen Position befindet, dieses die Öffnung in dem Begrenzungselement (23) und in dem Haltelement (35) vollständig gas-, dampf-, und/oder flüssigkeitsfest verschließt und zusätzlich von dem Halteelement (25) derart in der oberen Position (24) gehalten wird, dass das kugelförmige Dichtelement (29) nur durch Beaufschlagung einer hinreichend großen Kraft von der oberen Position (24) zu der unteren Position (28) oder von der unteren Position (28) zu der oberen Position (24) verschoben werden kann,
und wobei das Halteelement einen Durchfluss für Gase und Flüssigkeiten ausbildet, wenn sich das kugelförmige Dichtelement (29) in der unteren Position (28) befindet.

2. Filter nach Anspruch 1, wobei der Hohlraum des Körpers zylindrisch ist.

3. Filter nach Anspruch 1 oder 2, wobei das erste Abschlusselement (2) und das Begrenzungselement (23) mit dem Körper (6) einstückig ausgeführt sind.

4. Filter nach einem der vorangehenden Ansprüche, wobei die Beaufschlagung des kugelförmigen Dichtelements (29) mit Kraft zur Verschiebung von einer Position in die jeweils andere Position mittels Drucks erfolgen kann, welcher durch eine mit dem Entlüftungsventil in einem Kreislauf befindliche Pumpe erzeugt wird.

5. Filter nach einem der vorangehenden Ansprüche, wobei das kugelförmige Dichtelement (29) und / oder das Halteelement (25) aus einem elastischen Material ausgeführt sind.

6. Filter nach Anspruch 5, wobei das kugelförmige Dichtelement (29) und / oder das Haltelement (25) aus einem weichen Kunststoff oder Gummi ausgeführt sind.

7. Filter nach einem der vorangehenden Ansprüche, wobei das Material und die Abmessungen des Dichtelements (29) und der Durchmesser der Öffnung des Halteelements (25) so gewählt sind, dass die Kraft, welche zu beaufschlagen ist, um das Dichtelement von der oberen Position (24) in die untere Position (28) zu bringen, einem Druck entspricht, der größer ist, als die Druckschwankungen während des Betriebs eines an das Entlüftungsventil angeschlossenen Fluidkreislaufs.

8. Filter nach einem der vorangehenden Ansprüche, wobei das Entlüftungsventil in einer Entlüftungskappe (33) des Filters integriert ist.

9. Dialysierflüssigkeitskreislauf zur Zubereitung von Dialysierflüssigkeit mit einem Filter nach einem der vorangehenden Ansprüche.

## Claims

1. A filter for a dialysis fluid circulation, wherein the filter has a ventilating valve for ventilating the filter,
wherein the filter comprises:
- a body (6) having a cavity (8), a first opening (9) and a second opening (1),
- a first closing element (2), which is connected to the body (6) and has the second opening (1), which accommodates a hydrophobic membrane,
- a spherical sealing element (29) in the interior of the cavity, which is designed to float at the surface of a liquid and can be moved between a lower position (28) and an upper position (24),
- a closing element (27), which is connected to the body (6) and predefines the lower position (28),
- a limiting element (23), which predefines the upper position (22) and has an opening,
- a holding element (25) having an opening, which is arranged between the closing element (27) and the limiting element (23), such that when the sealing element (29) is in the upper position, it completely closes the opening in the limiting element (23) and in the holding element (35) in a gastight, vapor-tight and/or liquid-tight manner and is also held in the upper position (24) by the holding element (25) such that the spherical sealing element (29) can be displaced from the upper position (24) to the lower position (28) or from the lower position (28) to the upper position (24) only by applying a sufficiently great force,
and wherein the holding element forms a flow element for gasses and liquids when the spherical sealing element (29) is in the lower position (28).

2. The filter according to claim 1,
wherein the cavity of the body is cylindrical.

3. The filter according to claim 1 or 2,
wherein the first closing element (2) and the limiting element (23) are designed in one piece with the body (6).

4. The filter according to any one of the preceding claims,
wherein the spherical sealing element (29) is acted upon with force for displacement from one position into the other position by means of pressure generated by a pump in circulation with the ventilation valve.

5. The filter according to any one of the preceding claims,
wherein the spherical sealing element (29) and/or the holding element (25) is/are made of an elastic material.

6. The filter according to claim 5,
wherein the sealing element (29) and/or the holding element (25) is/are made of a soft plastic or rubber.

7. The filter according to any one of the preceding claims,
wherein the material and the dimensions of the sealing element (29) and the diameter of the opening of the holding element (25) are selected, so that the force to be applied to move the sealing element from the upper position (24) into the lower position (28) corresponds to a pressure that is greater than the pressure fluctuations during operation of a fluid circulation connected to the ventilation valve.

8. The filter according to any one of the preceding claims,
wherein the ventilation valve is integrated into a ventilation cap (33) on the filter.

9. A dialysis fluid circulation for preparation of dialysis fluid with a filter according to any one of the preceding claims.

## Revendications

1. Filtre pour circuit de liquide de dialyse, ce filtre présentant une soupape de purge pour la purge du filtre, et la soupape comprenant :
- un corps (6) doté d'une cavité (8), d'une première ouverture (9) et d'une seconde ouverture (1),
- un premier élément terminal (2) qui est connecté au corps (6) et présente la seconde ouverture (1) dans laquelle une membrane hydrophobe et reçue,
- un élément d'étanchéité de forme sphérique (29) situé à l'intérieur de la cavité et qui est conçu pour flotter dans un liquide à la surface et qui est mobile entre une position inférieure (28) et une position supérieure (24),
- un élément terminal (27) qui est connecté au corps (6) et prescrit la position inférieure (28),
- un élément de limitation (23) qui prescrit la position supérieure (22) et présente une ouverture,
- un élément de retenue (25) doté d'une ouverture qui est pratiquée entre l'élément terminal (27) et l'élément de limitation (23) de manière à ce que l'élément d'étanchéité (29) se trouve dans la position supérieure, à ce que celui-ci ferme l'ouverture de l'élément de limitation (23) et de l'élément de retenue (35) de manière entièrement étanche au gaz, à la vapeur et/ou au liquide et soit en outre maintenu par l'élément de retenue (25) dans la position supérieure (24) de manière à ce que l'élément d'étanchéité de forme sphérique (29) ne puisse être décalé que par l'application d'une force suffisamment importante depuis la position supérieure (24) vers la position inférieure (28) ou depuis la position inférieure (28) vers la position supérieure (24),
et l'élément de retenue permettant le passage de gaz et de liquide lorsque l'élément d'étanchéité de forme sphérique (29) se trouve dans la position inférieure (28).

2. Filtre selon la revendication 1, dans lequel la cavité du corps est cylindrique.

3. Filtre selon la revendication 1 ou 2, dans lequel le premier élément terminal (2) et l'élément de limitation (23) sont réalisés en une pièce avec le corps (6).

4. Filtre selon une des revendications précédentes, dans lequel la sollicitation de l'élément d'étanchéité de forme sphérique (29) avec de la force pour le décaler d'une position vers l'autre position respective peut se faire par une pression qui est générée par une pompe se trouvant dans un circuit avec la soupape de purge.

5. Filtre selon une des revendications précédentes, dans lequel l'élément d'étanchéité de forme sphérique (29) et/ou l'élément de retenue (25) sont réalisés dans un matériau élastique.

6. Filtre selon la revendication 5, dans lequel l'élément d'étanchéité de forme sphérique (29) et/ou l'élément de retenue (25) sont réalisés en plastique mou ou en caoutchouc.

7. Filtre selon une des revendications précédentes, dans lequel le matériau et les dimensions de l'élément d'étanchéité (29) et le diamètre de l'ouverture de l'élément de retenue (25) sont sélectionnés de manière à ce que la force appliquée pour amener l'élément d'étanchéité de la position supérieure (24) à la position inférieure (28) corresponde à une pression qui est supérieure aux variations de pression pendant le fonctionnement d'un circuit de fluide connecté à la soupape de purge.

8. Filtre selon une des revendications précédentes, dans lequel la soupape de purge est intégrée dans un capot de purge (33) du filtre.

9. Circuit de liquide de dialyse pour la préparation de liquide de dialyse avec un filtre selon une des revendications précédentes.
